# EUROPEAN PATENT APPLICATION

(11) **EP 2 923 678 A1**
(43) Date of publication of application: **30.09.2015**
(21) Application number: 14162230.8
(22) Date of filing: 28.03.2014
(51) Int. Cl.: A61F 5/02, A41D 13/00

(54) **Clothing article**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A clothing article includes a torso element (10) having a right shoulder portion (11) and a left shoulder portion (12) and a back portion (13), a right elastic piece (20) is disposed on the right shoulder portion (11) of the torso element (10) for engaging with a right shoulder of a user, a left elastic piece (21) provided on the left shoulder portion (12) of the torso element (10) for engaging with a left shoulder of the user, and a lower elastic piece (22) disposed on the back portion (13) of the torso element (10) and coupled between the right and the left elastic pieces (20, 21) for pulling the right and the left shoulders of the user toward each other and for correcting a posture of the user and for preventing the user from heading down.

## Description

The invention relates to a clothing article.

Typical clothing articles comprise a body formed of knitting fabric, for being worn by the user.

However, the typical clothing articles may not correct or improve the posture of the user.

In order to solve this technical problem the invention provides a clothing article including a device for correcting the posture as indicated in the claims.

The clothing article includes a device for correcting the posture and for preventing the user from heading down

In the drawings:
FIGS. 1, 3 are front perspective views of an embodiment of a clothing article according to the invention;
FIGS. 2, 4 are rear perspective views of the clothing article;

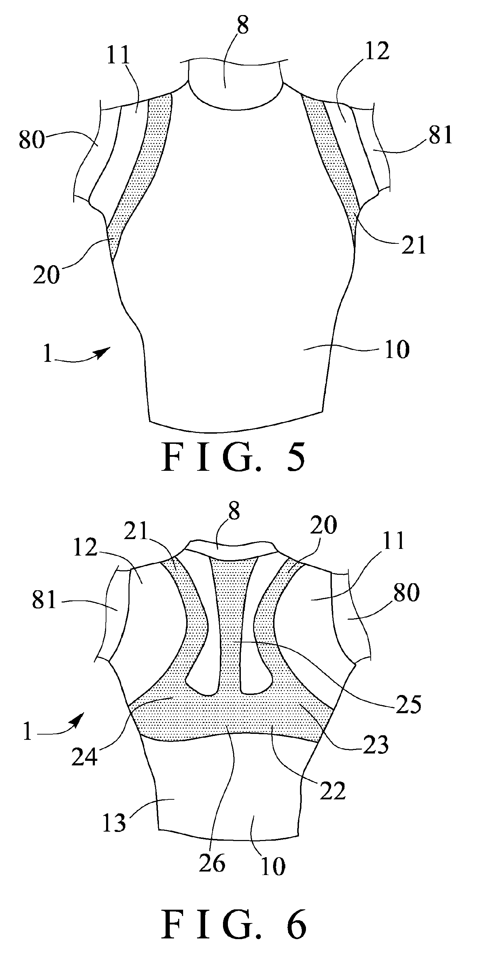

FIG. 5 is a partial front plan view of the clothing article; and
FIG. 6 is a partial rear plan view of the clothing article.

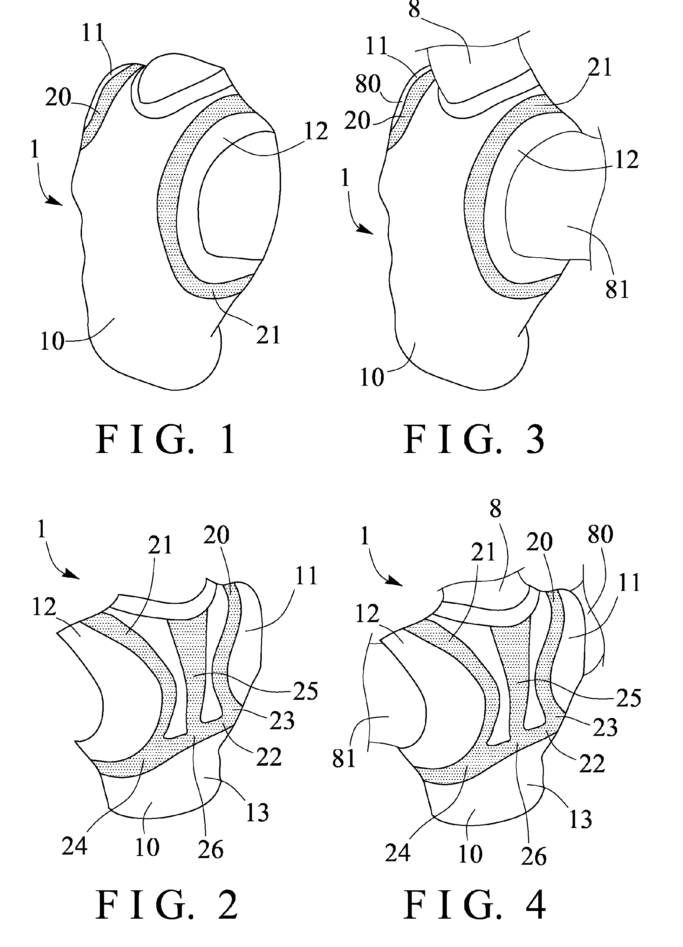

Referring to FIGS. 1 and 2, a clothing article 1 comprises a torso element 10, and a right shoulder portion 11 and a left shoulder portion 12 disposed beside the torso element 10, a right elastic piece 20 provided on the right shoulder portion 11 for engaging with the right shoulder 80 of the user 8, and a left elastic piece 21 provided on the left shoulder portion 12 for engaging with the left shoulder 81 of the user 8, and a lower elastic piece 22 provided on the back portion 13 and coupled between the lower portions 23, 24 of the right and the left elastic pieces 20, 21 for pulling the shoulders 80, 81 of the user 8 toward each other and for correcting the posture and for preventing the user from heading down. The clothing article 1 may include a middle elastic piece 25 provided on the middle of the back portion 13 of the torso element 10 and coupled to the middle portion 26 of the lower elastic piece 22.

The torso element 10 including the right shoulder portion 11 and the left shoulder portion 12 may be shaped like a tight shirt for covering the torso in the upper part of the body of a person. The right elastic piece 20 and its lower portion 23 may form a loop around the right shoulder joint of a person. The left elastic piece 21 and its lower portion 24 may form a loop around the left shoulder joint of a person. The lower portions 23, 24 may be connected by the middle portion 26 extending horizontally across the body. The middle elastic piece 25 may extend vertically upwards from the middle portion 26 to a collar of the torso element 10, said collar extending around a persons neck.

The elastic pieces 20 to 26 may be formed as elastic straps. These elastic straps may be integrated into the fabric of the torso element 10. Alternatively, the may be attached to the fabric of the torso element 10. Further, some of the straps may be integrated while others are attached to the torso element 10.

## Claims

1. A clothing article comprising
a torso element (10) including a right shoulder portion (11) and a left shoulder portion (12) and a back portion (13),
**characterised in that**:
a right elastic piece (20) is provided on the right shoulder portion (11) of the torso element (10) for engaging with a right shoulder of a user,
a left elastic piece (21) is provided on the left shoulder portion (12) of the torso element (10) for engaging with a left shoulder of the user, and
a lower elastic piece (22) is provided on the back portion (13) of the torso element (10) and coupled between the right and the left elastic pieces (20, 21) for pulling the right and the left shoulders of the user toward each other and for correcting a posture of the user.

2. The clothing article as claimed in claim 1, **characterised in that** the lower elastic piece (22) is coupled between lower portions (23, 24) of the right and the left elastic pieces (20, 21).

3. The clothing article as claimed in claim 1 or 2, **characterised in that** a middle elastic piece (25) is provided on the back portion (13) of the torso element (10) for further pulling the back portion (13) of the user.

4. The clothing article as claimed in claim 3, **characterised in that** the middle elastic piece (25) is coupled to an intermediate portion (26) of the lower elastic piece (22).

5. The clothing article as claimed in one of claims 1 to 4, **characterised in that** the right elastic piece (20) and its lower portion (23) are configured to form a loop around the right shoulder joint of a person and the left elastic piece (21) and its lower portion (24) are configured to form a loop around the left shoulder joint of a person.
